# EUROPEAN PATENT SPECIFICATION

(11) **EP 1 737 398 B1**
(45) Date of publication and mention of the grant of the patent: **01.02.2012**
(21) Application number: 05735271.8
(22) Date of filing: 14.04.2005
(51) Int. Cl.: A61F 5/00

(54) **SPLINT OR SUPPORT WITH QUICK LOCATION TECHNIQUE**
SCHIENE ODER STÜTZE MIT SCHNELLER LOKALISIERUNGSTECHNIK
ATTELLE OU SUPPORT A TECHNIQUE DE POSITIONNEMENT RAPIDE

(30) Priority: 20.04.2004 US 828744
(43) Date of publication of application: 03.01.2007
(73) Proprietor: ROYCE MEDICAL COMPANY, Camarillo, California 93012 (US)
(72) Inventor: GRIM, Tracy, E., Bixby, OK 74008 (US); HENDERSON, Wendy, Ventura, CA 93003 (US); IGLESIAS, Joseph, M., Newbury Park, CA 91320 (US); CAMPOS, Michael, Sylmar, CA 91342 (US); DOUBLEDAY, Walter, Jupiter, FL 33488 (US); SPEAKES, Kelly, M., Woodland Hills, CA 91367 (US)
(74) Representative: Donné, Eddy
(86) International application number: PCT/US2005/012553
(87) International publication number: WO 2005/105002

(56) References cited:
- US-A- 5 755 678
- US-A- 6 106 492
- US-A- 6 139 513

## Description

### BACKGROUND OF THE INVENTION

This invention relates to orthopaedic splints or supports.

In the field of hardenable orthopaedic splints and supports, the splints are nonnally rectangular in shape and are held on to the anatomy by a finishing tape of some kind wrapped along the entire length of the splint. To function properly, both the splint and finishing tape are soft and supple in order to conform to the contours of the anatomy.

Examples of such splints are known from for instance documents US 5.755.678 and US 6.106.492. During application of the splint, it is necessary that the limb be held in a variety of positions. Frequently, lengthy splints must be used to properly immobilize the injured limb, and gravity causes one end or the other of the splint to fall away from the anatomy or merely shift away from the proper position. If one person is attempting to apply the splint, it is difficult to maintain the appropriate position for each particular injury during hardening. Usually it is necessary to ask for additional assistance to ensure proper application. More particularly, an extra set of hands is required to keep the splint in the desired location on the patient's anatomy until the outer securing or immobilization means has been applied.

### SUMMARY OF THE INVENTION

The present invention therefore relates to an efficient splint or support comprising: an orthopaedic blank impregnated with hardenable material and having an outer surface covered with hook receivable material; a primary tacking arrangement for holding said blank in place on the injured part of the patient's anatomy in a manner that still allows adjustment of said blank with respect to the anatomy and secondary holding arrangements provided over said primary tacking arrangement for functionally securing the blank in place on the patient after the blank has been activated and properly mounted on the patient; whereby the primary tacking arrangement includes at least one strip of a non-woven material having opposed end portions, and hook type patches secured on the opposed end portions, the non-woven material is adapted to receive the hook type patches, wherein the strip is stretchable, tearable, low profile and of insufficient strength to rigidly immobilize the splint for long-term use, the hook receivable material on the outer surface of the blank is adapted to receive the hook type patches of the strip; whereby the primary tacking arrangement is removable and repositionable at any location along an axial length of the blank; and whereby the splint or support may be easily and property mounted on the patient.

In accordance with the present invention a splint or support is provided with primary tacking arrangements to locate the splint in place, allowing the physician or technician the use of two free hands for quickly and easily adjusting the position of the splint blank if necessary without removing or loosening said arrangements. The user then applies secondary, more supportive securing arrangements to effectively hold the splint or support to the injured part of the anatomy.

In accordance with one illustrative implementation of the invention, the primary tacking arrangements may involve mating hook and loop type material such as VELCRO® type inserts or pads, or hook type patches used with a splint covering of unbroken loop (UBL) or other hook receivable material on the surface of the splint. On this regard the entire hook receivable surface acts as the loop portion of the hook and loop fastening arrangements.

In addition, with the splint or support normally being longitudinal in extent, laterally extending securing extensions may be provided with hook and loop, adhesive or other securing arrangements at the outer ends thereof. These lateral extensions may be formed as part of the hardenable portion of the splint, or as part of one layer thereof, or may be separate straps or the like, secured to the splint blank.

The secondary or "functional" securing arrangements may be in the form of a stretchable wrap such as an ACE® bandage, or may be straps, where the said arrangements are of sufficient strength to firmly hold the splint to the injured part of the anatomy during regular usage for extended periods of time.

Other features which may be included in implementation of the invention may involve the following:
1. The use of "spacer" type double knit material for the splint.
2. The use of an outer or secondary support such as an exo-skeleton type support, with associated straps serving as the functional securing arrangements.
3. The use of non-rectangular splints, with laterally extending areas forming the primary securing arrangements.
4. The use of a roll of splinting material, with lateral extensions spaced along its length.
5. The use of splint blanks with a thumbhole or another web space locater forming a part of the assembly.

In the field of splinting it is normally desirable to fully secure the splint or cast so that it will not come off or shift location during the normal course of daily activities. The primary holding or splint locating arrangements in the present invention are used when the splint blank is flexible and is being initially applied. These primary securing arrangements. may be considered to be temporary "tacking" or locating arrangements, as they permit easy adjustment of the splint position, and are usually of insufficient strength to rigidly immobilize the splint for long term use by the patient.

Accordingly, when the term "tacking" is used in the present specification and claims, reference is being made to the primary holding arrangements which are of insufficient strength to fully secure the splint; and subsequent securing arrangements are normally required in addition to the "tacking." The "tacking" is implemented by hook and loop fastening arrangements and functional securing of the splint may be accomplished by lamination of the layers when the edges of hardenable material are overlapped, by wrapping with flexible tape, such as an ACE® bandage tape, by straps, or by any other securing arrangement of sufficient strength to properly immobilize the splint to the injured portion of the anatomy.

It is further noted that the use of a layer of UBL or hook-receivable material has the additional advantage of restricting the flow of the hardenable material, as set forth in, for example, U.S. Patent No. 6,824,522 (Henderson, et al.), titled "Hardenable Orthopaedic Supports,".

In accordance with a method illustrating certain aspects of the invention, a splint or support is formed with sheet material impregnated with hardenable material, and with one or more primary laterally extending tacking extensions. The hardenable material is activated and the splint or support is held to the anatomy by engaging the laterally extending extensions; and the position and/or configuration of the impregnated sheet material is adjusted prior to the hardening of the hardenable sheet material while it is being loosely held by the tacking extensions; and the adjusted position is maintained while the hardening is accomplished.

Other objects, features and advantages will become apparent from a consideration of the following detailed description and the accompanying drawings.

### BRIEF DESCRIPTION OF THE DRAWINGS

Figure 1 is perspective view of a short arm splint embodiment illustrating the principles of the invention; the straps 18, 20, 22 do not represent the invention.
Fig. 2 is a partial cross-sectional view of part of the assembly of Fig. 1 prior to application; the straps 18, 20, 22 do not represent the invention.
Fig. 3 shows a splint roll with laterally extending tacking arrangements;

### DETAILED DESCRIPTION OF THE PREFERRED EMBODIMENTS

While the specification describes particular embodiments of the present invention, those of ordinary skill can devise variations of the present invention without departing from the inventive concept.

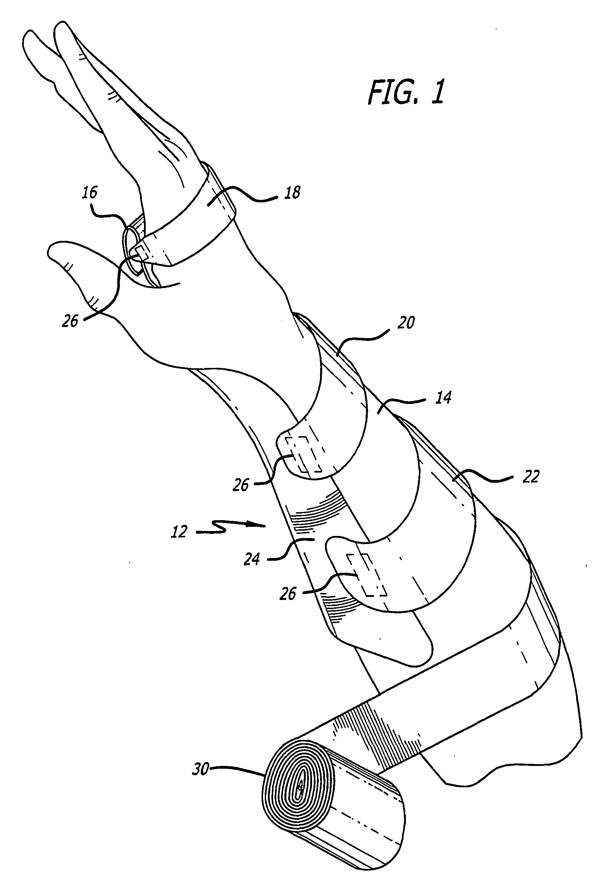

Referring more particularly to the drawings, Fig. 1 shows a splint or brace 12 mounted on the forearm 14 of a patient. The main part of the brace 12 is optionally folded over at reference numeral 16 at the palm of the patient's hand, and strap 18 extends over the back of the hand. The straps 18, 20 and 22 are secured to or are part of the main part 24 of the splint, and are provided with hook type fastener 26 on the underside of straps 18, 20 and 22 as shown in Fig. 1. The areas of the splint 12 underlying patches 26 are of hook receivable type material, so the straps 18, 20 and 22 are held or "tacked" in place as the splint 12 is applied. With the splint being held onto the forearm in an adjustable manner, the physician or medical technician can readily position the splint to the precise desired location, without the assistance of additional medical personnel.

The splint location is subsequently immobilized by wrapping the flexible, resilient tape 30 around the forearm and splint. This tape 30 may be of the type sold under the ACE® trademark.

Incidentally, relative to the hook receivable type material underlying the hook type patches 26, it may either be in the form of hook receivable type patches secured to the splint 12 in selected areas; or alternatively, the entire splint may be covered with a layer of unbroken loop (UBL) or other hook receivable material, so that the hook type patches will engage and secure to the splint 24 at any convenient location.

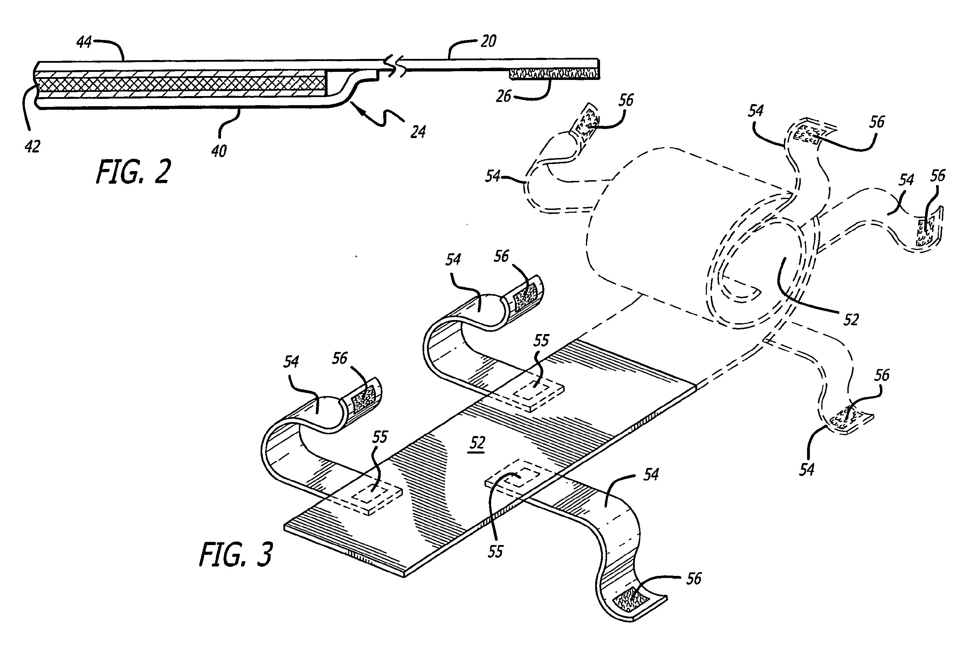

Furthermore, instead of VELCRO® type fasteners, adhesive, snaps, or hooks, for example, could be employed for tacking the straps 18, 20 and 22 in place. Figure 2 is a schematic cross-sectional view of a part of the assembly of Fig. 1 prior to application. In Fig. 2, the main portion 24 of the splint 12 includes an inner padding layer 40, and a central layer of hardenable material 42 preferably made of a double knit spacer material. The double knit spacer material includes upper and lower woven or knit layers, with an integral matrix of fibers or filaments interconnecting the upper and lower layers. An additional layer of unbroken loop (UBL) or hook receivable material 44 is adjacent to the upper surface of the spacer material 42. The strap 20 may be formed of separate material, or may be a continuation of any one of the layers of the main body 24 of the splint blank. It may also be either permanently attached or removably attached, using VELCRO®, hooks, etc. At the outer end of strap 20 is a patch 26 of hook type material for securing to underlying hook receivable type material 44.

The hardenable layer 42 is preferably impregnated with a water activatable material such as urethane. The layer of hook receivable or UBL material 44 may inhibit the transfer of the water activatable material to the outer surface of the splint.

Figure 3 shows a continuous roll of a splinting material 52 with laterally outwardly extending tacking arrangements 54 having patches 56 of hook type material located at the ends thereof. The main body of the splint roll 52 consists of at least one layer of material impregnated with a water activatable material. The outer surface of the splint roll 52 may be covered with hook receivable material or may be provided with patches of hook receivable type material for tacking engagement with the hook type patches 56. The tacking strips 54 may be in the form of separate strips with hook type patches 55 at the inner ends of strips 54 as well as on the outer ends of strips 54 where hook type patches 56 are provided. This gives flexibility in the location of the tacking arrangements of the splint onto the anatomy. A splint or support may be cut from the roll at any desired length to form a blank of the desired size.

The preferred splint roll configuration is an inner activatable material of at least one layer with an outer covering material on one side and a padding layer on the other. The outer covering is preferably made from a hook receivable material, such as a UBL material, and the padding may consist of at least one layer of either a double knit spacer material, foam, or a nonwoven material. Throughout the length of the splint are tacking arrangements spaced at equal intervals. The extensions may have hook type arrangements on both ends so that they would be removable and movable with respect to the splint assembly. The tacking arrangements are made of a stretchable material and have a configuration that would ensure proper securing yet not cause any discomfort to the end user. An acceptable material to use would be a nonwoven because of its cost effectiveness and low profile. The splint can be cut to usable lengths prior to packaging or may be provided in long lengths and may be cut to the appropriate size immediately prior to application.

In the case of all of the embodiments as disclosed in this specification, the construction and alternatives as disclosed herein may be used in each of the constructions intended for specific application. Thus, for example, after cutting off a length of the splint roll 52, activating it, and tacking it in place, the splint may be wrapped with a flexible wrap, to functionally secure the splint.

In regard to the materials used for fabricating the various layers of the above mentioned splinting assemblies, the tacking arrangements are made from a nonwoven material. Nonwoven materials are typically very inexpensive and may be made to be hydrophobic which is very advantageous. The tacking materials may also be made of a knitted material, or foam. It is also beneficial if the tacking attachments are low profile, stretchable and tearable. The activatable layer can be composed of a double knit spacer material, multiple layers of a single knit material, foam laminate, nonwoven material, woven material, or any suitable material that will achieve sufficient strength upon hardening.

It is to be understood that the foregoing detailed description and the embodiments shown in the drawings are illustrative embodiments of the invention. Various alternatives and modifications may be made without departing from the scope of the invention.

Regarding the construction of the blanks, they may be of other forms than that shown in Fig. 2, and may include multiple layers of high strength material, for example, instead of a spacer material. Also, concerning hook receivable material, it may be in the form of patches of VELCRO® material, UBL material, or napped material, for examples. More generally the variations suggested by any of the drawings or related description are applicable to the other embodiments disclosed herein. Instead of being water hardenable, the splints may include two materials with arrangements for combining the materials to initiate hardening. Accordingly, the present specification is not limiting the invention precisely as described in detail hereinabove and shown in the drawings.

While the specification describes particular embodiments of the present invention, those of ordinary skill can devise variations of the present invention without departing from the inventive concept.

## Claims

1. An efficient splint or support (52) comprising: an orthopaedic blank (52) impregnated with hardenable material and having an outer surface covered with hook receivable material; a primary tacking arrangements (54) for holding said blank (52) in place on the injured part of the patient's anatomy in a manner that still allows adjustment of said blank with respect to the anatomy; and secondary holding arrangements (30) provided over said primary tacking arrangement (54) for functionally securing the blank (52) in place on the patient after the blank (52) has been activated and properly mounted on the patient; **characterized in that** the primary tacking arrangement (54) includes at least one strip of a non-woven material having opposed end portions, and hook type patches (55,56) secured on the opposed end portions, the non-woven material is adapted to receive the hook type patches, wherein the strips is stretchable, tearable, low profiles and of insufficient strength to rigidly immobilize the splint (52) for long-term use, the hook receivable material on the outer surface of the blank (52) is adapted to receive the hook type patches (55,56) of the strip; **in that** the primary tacking arrangement (54) is removable and repositionable at any location along an axial length of the blank (52); and **in that** the splint or support (54) may be easily and properly mounted on the patient.

2. An efficient splint or support according to claim 1, **characterized in that** said blank (52) is formed of double knit spacer type material.

3. An efficient splint or support according to claim 1 or 2, **characterized in that** said secondary holding arrangements (30) comprise an exo-skeletal structure overlying said blank (52).

4. An efficient splint or support according to claim 3, charactereized in that said strip is non-rectangular in shape.

5. An efficient splint or support according to claim 1 **characterized in that** said orthopaedic blank (52) has a padding layer on at least one side.

6. An efficient splint or support according to claim 1 **characterized in that** said secondary holding arrangements (30) include a resilient, flexible, stretchable tape.

## Patentansprüche

1. Effiziente Schiene oder Stütze (52), umfassend: einen orthopädischen Zuschnitt (52), der mit aushärtbarem Material imprägniert ist und eine Außenseite aufweist, die mit Material, das Häkchen aufnehmen kann, bedeckt ist; eine primäre Anheftanordnung (54) zum Festhalten besagten Zuschnitts (52) an seinem Platz an dem verletzten Teil der Anatomie des Patienten auf eine Weise, die noch ein Anpassen besagten Zuschnitts in Bezug auf die Anatomie gestattet; und sekundäre Halteanordnungen (30), die über besagter primärer Anheftanordnung (54) vorgesehen sind, um den Zuschnitt (52) funktionell an dem Patienten an seinem Platz zu halten, nachdem der Zuschnitt (52) aktiviert und fachgerecht an dem Patienten montiert wurde; **dadurch gekennzeichnet, dass** die primäre Anheftanordnung (54) mindestens einen Streifen aus einem Vliesmaterial beinhaltet, der entgegengesetzte Endteile aufweist, sowie an den entgegengesetzten Endteilen gesicherte Häkchenpflaster (55, 56); wobei das Vliesmaterial dazu eingerichtet ist, die Häkchenpflaster aufzunehmen, wobei der Streifen dehnbar, abreißbar, kleinformatig und von nicht ausreichender Stärke ist, um die Schiene (52) für längerfristigen Gebrauch starr zu immobilisieren, wobei das Material, das Häkchen aufnehmen kann, an der Außenseite des Zuschnitts (52) dazu eingerichtet ist, die Häkchenpflaster (55, 56) des Streifens aufzunehmen; dadurch, dass die primäre Anheftanordnung (54) abnehmbar und an gleich welcher Stelle entlang einer axialen Länge des Zuschnitts (52) umpositionierbar ist; und dadurch, dass die Schiene oder Stütze (54) mühelos und fachgerecht an dem Patienten angebracht werden kann.

2. Effiziente Schiene oder Stütze nach Anspruch 1, **dadurch gekennzeichnet, dass** besagter Zuschnitt (52) aus doppelt gewirktem Abstandsmaterial geformt ist.

3. Effiziente Schiene oder Stütze nach Anspruch 1 oder 2, **dadurch gekennzeichnet, dass** besagte sekundäre Halteanordnungen (30) eine über besagtem Zuschnitt (52) liegende Außenskelettstruktur umfassen.

4. Effiziente Schiene oder Stütze nach Anspruch 3, **dadurch gekennzeichnet, dass** besagter Streifen eine nicht rechteckige Form aufweist.

5. Effiziente Schiene oder Stütze nach Anspruch 1, **dadurch gekennzeichnet, dass** besagter orthopädischer Zuschnitt (52) eine Polsterschicht an mindestens einer Seite aufweist.

6. Effiziente Schiene oder Stütze nach Anspruch 1, **dadurch gekennzeichnet, dass** besagte sekundäre Halteanordnungen (30) ein elastisches, flexibles, dehnbares Band beinhalten.

## Revendications

1. Gouttière ou support efficace (52) comprenant une ébauche orthopédique (52) imprégnée avec une matière durcissable et possédant une surface externe recouverte d'une matière apte à recevoir un crochet ; un arrangement d'attachement primaire (54) pour maintenir ladite ébauche (52) en place sur la partie blessée de l'anatomie du patient d'une manière qui permet toujours le réglage de ladite ébauche par rapport à l'anatomie ; et des arrangements de maintien secondaire (30) qui sont prévus par-dessus ledit arrangement d'attachement primaire (54) pour fixer de manière fonctionnelle l'ébauche (52) en place sur le patient une fois que l'ébauche (52) a été activée et montée comme il se doit sur le patient ; **caractérisé en ce que** l'arrangement d'attachement primaire (54) englobe au moins une bande d'un non-tissé possédant des portions terminales opposées, et des pièces en forme de crochets (55, 56) fixées aux portions terminales opposées, le non-tissé étant conçu pour recevoir les pièces en forme de crochets, la bande étant extensible, déchirable, discrète et possédant une résistance insuffisante pour immobiliser de manière rigide la gouttière (52) pour une utilisation à long terme, la matière apte à recevoir un crochet sur la surface externe de l'ébauche (52) étant conçue pour recevoir les pièces en forme de crochets (55, 56) de la bande ; **en ce que** l'arrangement d'attachement primaire (54) est amovible et peut être repositionné à n'importe quel endroit sur la longueur axiale de l'ébauche (52) ; et **en ce que** la gouttière ou le support (52) peut être monté aisément et de manière adéquate sur le patient.

2. Gouttière ou support efficace selon la revendication 1, **caractérisé en ce que** ladite ébauche (52) est réalisée sous la forme d'une matière de type écarteur à tricot double.

3. Gouttière ou support efficace selon la revendication 1 ou 2, **caractérisé en ce que** lesdits arrangements de maintien secondaire (30) comprennent une structure exo-squelettique recouvrant ladite ébauche (52).

4. Gouttière ou support efficace selon la revendication 3, **caractérisé en ce que** ladite bande possède une configuration non rectangulaire.

5. Gouttière ou support efficace selon la revendication 1, **caractérisé en ce que** ladite ébauche orthopédique (52) possède une couche de rembourrage sur au moins un côté.

6. Gouttière ou support efficace selon la revendication 1, **caractérisé en ce que** lesdits arrangements de maintien secondaire (30) englobent un ruban résilient, flexible, extensible.
